(19) Europäisches Patentamt
European Patent Office
Office européen des brevets

(11) Publication number: 0 267 626
B1

(12) # EUROPEAN PATENT SPECIFICATION

(45) Date of publication of patent specification: 30.01.91

(51) Int. Cl.$^5$: C 07 C 43/12, C 07 C 41/06

(21) Application number: 87116811.8

(22) Date of filing: 13.11.87

(54) **Process for the preparation of fluorohalogenated ethers starting from fluorooxy compounds and halogenated olefins.**

(30) Priority: 14.11.86 IT 2234986

(43) Date of publication of application:
18.05.88 Bulletin 88/20

(45) Publication of the grant of the patent:
30.01.91 Bulletin 91/05

(84) Designated Contracting States:
DE FR GB NL

(56) References cited:
EP-A-0 201 871
GB-A-2 148 286

(73) Proprietor: AUSIMONT S.r.l.
31, Foro Buonaparte
I-20100 Milano (IT)

(72) Inventor: Guglielmo, Giorgio, Dr.
89, via A.Gramsci
I-30035 Mirano Venezia (IT)
Inventor: Gambaretto, Gian Paolo, Dr.
32/22, via Turazza
I-35100 Padova (IT)

(74) Representative: Barz, Peter, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

EP 0 267 626 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an improved process for obtaining fluorohalogenated ethers.

The fluorohalogenated ethers prepared according to this process are in particular suitable to be halogenated to give the corresponding perfluorovinylethers.

It is known to react the fluorooxy compounds in gaseous phase, at low temperature, with halogenated olefins to obtain the fluorohalogenated ethers of the above mentioned type (see Italian Patent Application 20781 A/85).

It is well known that fluorooxy compounds having a number of carbon atoms more than 1 are very explosive and they are very difficult to deal with.

Italian Patent Application 20781 A/85 describes a process in which the fluorooxy compound is continuously fed in gaseous phase in order to prepare in a continuous way fluorohalogenated ethers.

The disadvantage of this process resides in the fact that the yield for fluorooxy compounds containing more than two carbon atoms are unsatisfactory.

Due to the fact that the latter fluorooxy compounds are very explosive the teaching of the prior art is very poor.

GB-A-2 148 286 discloses a process for the preparation of chlorotrifluoroethylene telomers which comprises adding fluorooxy-trifluoromethane to a solution of a trifluorochloroolefin in an inert solvent. This process results in the formation of mixtures of telomers having different molecular weight, owing to the addition of two or more olefin units to the molecule of the fluorooxy compound.

An object of the present invention is to provide an improved process for the preparation of fluorohalogenated ethers in a continuous manner by using fluorooxy compounds having more than two carbon atoms.

Thus, the present invention relates to an improved process for preparing fluorohalogenated ethers having the general formula:

$$(R)_nC(F)_m\text{-O-CAF-CA'}F_2 \qquad (1)$$

wherein A an A′ are equal or different and are selected from chlorine and bromine, R is a $C_{1-20}$ alkyl radical or a cycloalkyl, aromatic, heterocyclic or polyether radical containing up to 20 carbon atoms, said radicals being partially or wholly halogenated with bromine, chlorine, iodine and/or fluorine, n is an integer having a value of 1 or 2, m is an integer equal to 3-n, it being understood that the value n=2 comprises the compounds wherein C belongs to a cyclic ring.

The process is based on the reaction between a fluorooxy compound of the general formula: $(R)_nC(F)_m$-OF with an olefin CAF=CA′F, wherein the symbols R, A, A′ n and m have the above-specified meaning, the reaction being carried out in liquid phase, at a temperature of from −150 to 0°C, preferably −40 to −100°C.

The process is characterized in that the fluorooxy compound is continuously fed into the reactor, in form of a solution in an inert solvent, at a concentration lower than 50% by weight. The mentioned solution may be obtained continuously by contacting the fluorooxy compound continuously fed in gaseous form, preferably diluted with an inert gas, with the reaction inert solvent.

As inert gaseous diluent of the starting fluorooxy compounds, the same reaction solvent may be used provided that it is in gaseous form in the compositions in which the fluorooxy compound is supplied. The halogenated olefins must always be present in the reaction phase in excess on the fluorooxy compound. The halogenated olefin may be fed all at the beginning into the reactor in liquid form, optionally diluted with an inert solvent which may be the same solvent used for the fluorooxy compound to be dissolved. Alternatively, also the olefin may be continuously fed.

Solvents suitable for the reaction are, in particular, chlorofluorocarbons, perfluorocarbons and perfluoroethers or perfluoropolyethers.

In the process according to the invention, the fluorooxy compound coming directly from the reactor of the synthesis of the same, in gaseous form, can be advantageously used. In fact, the inert diluents used in the reaction for the synthesis of the fluorooxy compound starting from fluorine and acyl fluoride can be compatible and suitable also for the present process.

Examples of perfluorohalogenated ethers which may be prepared by the process according to the invention are as follows:

$CF_3$-$CF_2$-O-CClF-$CClF_2$
$CClF_2$-$CF_2$-OCClF-$CClF_2$
$CCl_2F$-$CF_2$-O-CClF-$CClF_2$
$CCl_3$-$CF_2$-O-CClF-$CClF_2$
$CBrF_2$-$CF_2$-O-CClF-$CClF_2$
$CF_3$-O-$CF_2$-$CF_2$-O-CClF-$CClF_2$
$C_2F_5$-O-$CF_2$-$CF_2$-O-CClF-$CClF_2$
$(CF_3)_2$ CF-O-CClF-$CClF_2$.

The following examples are given only to illustrate the possible performance of the process according to the invention.

Example 1

A gaseous stream of fluorooxyperfluoroethane obtained by reacting trifluoroacetylfluoride and elemental fluorine fed separately into a catalytic reactor in the presence of ALGOFLON A114® (dichlorotetrafluoroethane) in the gaseous phase, contains 20% by volume of $C_2F_5OF$ and 80% of $C_2F_4Cl_2$.

This gaseous stream is cooled in a glass condenser externally cooled to −80°C with a flow of 18.7 Nl/h and is for the most part condensed. The thus obtained solution is added dropwise into a reactor cooled to −80°C containing a

strongly agitated solution of symmetric difluoroethylene (230 g) in 600 g of ALGOFLON A12® ($CF_2Cl_2$).

After 10 hours the fed perfluorooxy compound is equivalent to 95% of the olefin. The feeding is interrupted and the liquid in the reactor is distilled off.

A fraction of product boiling at 58 — 60°C (356 g; yield 80%) is recovered and identified as $CF_3$-$CF_2$-O-CFCl-$CF_2$Cl by the mass spectrometry.

Example 2

A gaseous stream containing 20% by volume of chlorotetrafluorooxyethane $CF_2$Cl-$CF_2$-OF and 80% by volume of ALGOFLON A114® obtained as in the preceding example is cooled in a condenser cooled to −30°C wherein it condenses almost completely and the thus obtained solution is added dropwise into a strongly agitated reactor, externally cooled to −80°C and containing 200 g of olefin CFCl=CFCl dissolved in 500 g of ALGOFLON A114®.

After 20 hours, always keeping the flow of the gas at 7.8 Nl/h the fed fluorooxy compound is about 92% of the olefin. At this moment the feeding is interrupted, the content of the reactor is distilled off and 79 g of a fraction boiling at 90—95°C, is recovered, the 95% of which consists of the compound $CF_2$Cl-$CF_2$-O-CFCl-$CF_2$Cl identified by mass spectrometry.

**Claims**

1. A process for the preparation of fluorohalogenated ethers having the general formula:

$$(R)_nC(F)_m\text{-O-CAF-CA'}F_2$$

wherein A and A′ are equal or different and are selected frcm chlorine or bromine, R is a $C_{1-20}$ alkyl radical or a cycloalkyl, aromatic, heterocyclic or polyether radical containing up to 20 carbon atoms, said radicals being partially or wholly halogenated with bromine, chlorine, iodine and/or fluorine, n is an integer chosen from 1 or 2, m is an integer equal to 3—n, wherein the value n=2 comprises the compounds wherein C belongs to a cyclic ring, which process comprises reacting in liquid phase a fluorooxy compound $(R)_nC(F)_m$-OF with an olefin CAF=CA′F, at a temperature of from −150 to 0°C, characterized in that the fluorooxy compound is continuously fed in the reaction phase, in form of a solution with a concentration lower than 50% by weight in an inert solvent, the olefin being fed in the liquid state, optionally in an inert solvent, all at the beginning into the reactor or continuously, in such a manner to have always an excess of the olefin in the reaction phase.

2. The process according to claim 1, wherein a chlorofluorocarbon or a perfluorocarbon or a perfluoroether or a perfluoropolyether is used as inert solvent.

3. The process according to claim 1 or 2, wherein the reaction is carried out at a temperature of from −40°C to −100°C.

**Patentansprüche**

1. Verfahren zur Herstellung von fluorohalogenierten Ethern der allgemeinen Formel:

$$(R)_nC(F)_m\text{—O—CAF—CA'}F_2$$

worin A und A′ gleich oder verschieden und aus Chlor und Brom ausgewählt sind, R ein $C_{1-20}$-Alkylrest oder ein Cycloalkyl-, aromatischer, heterocyclischer oder Polyetherrest mit bis zu 20 Kohlenstoffatomen ist, wobei die genannten Reste teilweise oder vollständig mit Brom, Chlor, Jod und/oder Fluor halogeniert sind, n eine ganze Zahl, ausgewählt aus 1 oder 2, bedeutet, m eine ganze Zahl 3−n darstellt, wobei der Wert n=2 die Verbindungen umfaßt, in denen C zu einem cyclischen Ring gehört, welches Verfahren das Umsetzen einer Fluoroxyverbindung $(R)_nC(F)_m$—OF mit einem Olefin CAF=CA′F in flüssiger Phase bei einer Temperatur von −150°C bis 0°C umfaßt, dadurch gekennzeichnet, daß die Fluoroxyverbindung der Reaktionsphase kontinuierlich in Form einer Lösung in einem inerten Lösungsmittel mit einer Konzentration niedriger als 50 Gewichtsprozent zugeführt wird, wobei das Olefin im flüssigen Zustand, gegebenenfalls in einem inerten Lösungsmittel, dem Reaktor gänzlich zu Anfang oder kontinuierlich so zugeführt wird, daß immer ein Überschuß an Olefin in der Reaktionsphase vorhanden ist.

2. Verfahren nach Anspruch 1, worin ein Chlorfluorkohlenstoff, oder ein Perfluorkohlenstoff oder ein Perfluorether oder ein Perfluorpolyether als inertes Lösungsmittel verwendet wird.

3. Verfahren nach Anspruch 1 oder 2, worin die Reaktion bei einer Temperatur von −40°C bis −100°C durchgeführt wird.

**Revendications**

1. Un procédé pour la préparations d'éthers fluorohalogénés ayant la formule générale suivante:

$$(R)_nC(F)_m\text{—O—CAF—CA'}F_2 \quad (1)$$

dans laquelle:

les radicaux A et A′ sont identiques ou différents et sont choisis entre le chlore et le brome;

R est un radical alkyle en $C_{1-20}$ ou un radical cycloalkyle, aromatique, hétérocyclique ou polyéther contenant jusqu'à 20 atomes de carbone, lesdits radicaux étant en totalité ou en partie halogénés par du brome, du chlore, de l'iode et/ou du fluor;

n est un entier valant 1 ou 2;

m est un entier égal à 3−n, étant entendu que la valeur n=2 englobe les composés dans lesquels C appartient à un noyau cyclique,

procédé qui consiste à faire réagir, en phase liquide, un composé fluoro-oxy $(R)_nC(F)_m$—OF

avec une oléfine CAF=CA'F à une température de −150 à 0°C, caractérisé en ce que le composé fluoro-oxy est introduit en continu dans la phase réactionnelle sous forme d'une solution ayant une concentration inférieure à 50% en poids dans un solvant inerte, l'oléfine étant introduite dans le réacteur à l'état liquide, éventuellement dans un solvant inerte, soit en totalité au début, soit encore en continu, de façon à disposer toujours d'un excès de l'oléfine dans la phase réactionnelle.

2. Le procédé selon la revendication 1, dans lequel on utilise comme solvant inerte un chlorofluorocarbone ou un perfluorocarbone, ou encore un perfluoroéther ou un perfluoropolyéther.

3. Le procédé selon la revendication 1 ou 2 dans lequel la réaction est mise en oeuvre à une température de 40 à 100°C.